Europäisches Patentamt

European Patent Office

Office européen des brevets

(11) Numéro de publication: **0 289 366 B1**

## (12) FASCICULE DE BREVET EUROPEEN

(45) Date de publication de fascicule du brevet: **20.01.93** (51) Int. Cl.5: **A61K 7/48**, A61K 7/06

(21) Numéro de dépôt: **88400374.0**

(22) Date de dépôt: **19.02.88**

(54) **Produit comprenant un composé organique du silicium combiné avec une substance cosmétiquement active.**

(30) Priorité: **04.03.87 FR 8702912**

(43) Date de publication de la demande:
**02.11.88 Bulletin 88/44**

(45) Mention de la délivrance du brevet:
**20.01.93 Bulletin 93/03**

(84) Etats contractants désignés:
**DE GB NL**

(56) Documents cités:
**EP-A- 0 156 968     CH-B- 488 736**
**FR-A- 1 157 158     FR-A- 2 369 840**
**FR-A- 2 510 407     FR-A- 2 561 915**
**FR-M- 1 069**

(73) Titulaire: **Gueyne, Jean**
**Périgord 1 6 Lacets Saint Léon**
**Monte-Carlo(MC)**

Titulaire: **Seguin, Marie-Christine**
**Périgord 1 6 Lacets Saint-Léon**
**Monte-Carlo(MC)**

Titulaire: **Bondon, Jacques**
**4 Avenue Pasteur**
**F-06240 Beausoleil(FR)**

(72) Inventeur: **Gueyne, Jean**
**Périgord 1 6 Lacets Saint Léon**
**Monte-Carlo(MC)**
Inventeur: **Seguin, Marie-Christine**
**Périgord 1 6 Lacets Saint-Léon**
**Monte-Carlo(MC)**
Inventeur: **Bondon, Jacques**
**4 Avenue Pasteur**
**F-06240 Beausoleil(FR)**

(74) Mandataire: **Kohn, Armand c/o S.A. FEDIT-LORIOT ET AUTRES et al**
**CONSEILS EN PROPRIETE INDUSTRIELLE 38, avenue Hoche**
**F-75008 Paris(FR)**

## Description

La présente invention concerne un procédé pour bloquer le cheminement au niveau de la peau de complexes moléculaires d'organo-siliciés à action spécifique cosmétique, formulés ultérieurement dans des compositions cosmétiques. Elle vise notamment les cas où une molécule silicium organique est complexée d'une part à une substance capable de fixer le composé au niveau intra-cutané, d'autre part à une substance distinguant une spécificité révélée par le silicium organique dans le domaine cosmétique.

L'action des silanols, composés biologiquement actifs, est connue. Mais en cosmétique, ils présentent l'inconvénient d'être repris dans bien des cas par la microcirculation.

La présente invention a pour but la localisation intracutanée des dérivés ou des complexes organo-siliciés, leur permettant d'agir spécifiquement en réponse aux problèmes posés en cosmétique.

Grâce à l'invention, cette action du silicium est renforcée avec un déterminisme dans l'action limitée à la peau. Ainsi, le procédé de l'invention a-t-il pour effet la fixation de la substance active d'organo-silicium, recherchée pour son concept de traitement au niveau de la peau, empêchant pratiquement le passage de ces silanols-cosmétiques dans les tissus sousjacents.

Ce résultat est impératif pour répondre aux règlementations cosmétiques en vigueur (loi sur les cosmétiques) en adaptant l'action générale du silicium à un effet cosmétique particulier. Dans les documents de brevet FR-A-2 369 840 et FR-A-2 510 407 sont décrits des produits à base de silanol pour le traitement de la cellulite, c'est à dire de tissus formés sous la peau; il s'agit ainsi de compositions traversant la peau. Le document FR-A-2 561 915 décrit un produite hydratant constitué par un silanol et une substance polymère hydrosoluble, sans qu'aucune condition ne soit prévue pour éviter le passage du Si dans le tissu sous-jacent de la peau. Ce sont donc les avantages précités qu'apportent les nouveaux corps dénommés silicium-cosmétiques suivant l'invention.

Des composés constitués par des solutions ou/et des dispersions aqueuses d'un organo-silicié sont connus dans l'art.

Un silicium-cosmétique, suivant l'invention, est constitué par un complexe moléculaire, de type hydroxy-silanes de formule générale :

$$R_nSi(OR')_m(OR'')_p$$

où

$$n+m+p = 4$$

- R est un substituant organique sans aucune limitation, il peut être un alkyle ou alcényle, n est un nombre de 1 à 3 ;
- R' est un composé biologiquement actif, spécifique :
  R' apporte donc une spécificité à l'action du silicium ;
  le choix de R' est toujours fait d'une manière judicieuse.

R' est choisi parmi les différents corps chimiques qui seuls, en quantité importante ou en combinaison avec Si, peuvent produire sur la peau des effets cosmétiques, tels que par exemple amincissement, régénération, stimulation des peaux atones caractérisées par des tissus épidermiques distendus, manque de couleur, d'éclat, de tonus, réhydratation, rétablissement de la teneur en gras (action lubrifiante), activation de bronzage, protection contre les U.V., anticouperose, épaississement des peaux fines, antivergéture, régression des chéloïdes, antirides, action délipidante, antiséborrhéique, traitement des peaux jeunes à boutons, action calmante sur les épidermes irrités par rasage ou épilation, etc., action émoliante (ongles et corne des pieds), désodorisant, suppression des stases épidermiques à localisations diverses, cernes, poches sous les yeux, jambes lourdes, action durcissante des ongles, conditionneur pour cheveux, action stimulatrice avec rétablissement des équilibres sur la repousse des cheveux, régression de la canitie, protecteur et réparateur d'agressions divers (vent, froid, U.V., etc.). En général, les substances actives R' sont des composés organiques qui peuvent être porteurs, en particulier, d'une ou de plusieurs fonctions alcool, phénol, acide, amine ou aminoacide.

Ainsi, peut-on employer des substances telles que la théophylline et ses dérivés, caféine, l'acide acétyltyrosine, acide glycyrrhyzique, l'hydroxyproline, la sérine et autres acides aminés, alcool oléique, acide parahydroxycinnamique, acide lactique, acide pyrrolidone carboxylique, acide mannuronique, acide hyaluronique, ou bien d'autres, ces quelques substances étant citées seulement à titre non limitatif.

- R'' est une molécule dermatophile pouvant provoquer une réaction biochimique ou biophysique de

2

fixation capable de retenir aux différents niveaux de la peau l'ensemble du complexe, afin d'observer l'action cutanée, recherchée, sans reprise de tout ou partie du complexe par la microcirculation dermique. R'' est absorbable par la peau.

R'' complexée est un composé organique porteur d'une ou de plusieurs fonctions alcool, phénol, acide, amine, aminoacide. Il peut appartenir à un des groupes suivants.

1) Une macromolécule ; en particulier les macromolécules azotées ou glucidiques de type protidiques, lipoprotidiques, nucléoprotidiques, mucopolysaccharidiques, ou autres macromolécules mixtes, comportant des chaînes ou groupes avec des chaînes d'une autre nature.

Ces macromolécules peuvent être de synthèse ou naturelles, ou bien leurs produits d'hydrolyse contrôlée, c'est-à-dire dans le cas de protides, des protéines à chaînes plus courtes, polypeptides, cyclopeptides, peptides. A titre d'exemple on peut citer : albumine de l'oeuf, sérum de sang ou de lait, fibrine, gliadine, glutéine, myogène, mycélium de divers bactéries, élastine et les macromolécules résultant d'une hydrolyse ménagée, caséine, collagène, kératine, mucine, salmine, clupéine, zéine, A.D.N., A.R.N. acide pectique, pectines, acide alginique et autres, cités ici à titre d'exemples non limitatifs.

Rentrent également dans ce groupe les macromolécules à activités enzymatiques telles que : désoxyribonucléase, ribonucléase, collagénase, glucoronidase, etc.

2) Un polyphénol seul ou sous forme d'hétéroside plus ou moins polycondensé tel que catéchine, rutine, rutoside, esculétol et esculoside etc.

3) Un insaponifiable stérolique ou non, naturel ou de synthèse, animal ou végétal, tel qu'insaponifiable de soja, de luzerne, de beurre de karite, de carotte, de tomates, de noix, de ricin, d'avocat, tel que cholestérol, stigmastérol, sitostérol, caroténoïde, xanthophylle, tocophérol, déhydrocholestérols, pyridoxine, thiamine, c'est-à-dire l'ensemble des vitamines.

4) Un oligoélément métallique ou non, sous forme de sel d'acide minéral ou organique et de préférence d'un acide organique de haut poids moléculaire tel que pyrrolidone carboxylate de cuivre ou zinc, ou nickel, etc., hyaluronate de magnésium ou manganèse, ou potassium etc.; l'hémoglobine, le cytochrome, sans que cette liste soit limitative.

Ces silanols-cosmétiques peuvent être obtenus par transalcoxylation suivant l'équilibre :

$$R_nSi(OR')_{(4-n)} + R''OH \leftrightarrow$$
$$R_nSi(OR')_{(4-n-1)}(OR'') + R'OH$$

Cette réaction est réalisée dans un solvant organique, inerte vis-à-vis du siloxane (dépourvu de fonction alcool, amine primaire et secondaire, acide).

D'autre part, on peut préparer les produits suivant l'invention par complexation d'un alkyl silanetriol avec un mélange de composés organiques R'OH et R''OH où R' et R'' sont les substituants décrits plus haut. Cette complexation est déjà connue, elle peut être réalisée par mélange de solutions des réactifs concernés.

Lorsqu'un complexe a d'abord été formé avec un composé R', le silicium cosmétique, selon l'invention, peut être obtenu par transalcoxylation avec une molécule R'' si celle-ci est de la forme R''OH.

La réaction peut alors s'écrire :

$$R_nSi(OR')_{(4-n)} + mR''OH \rightarrow R_nSi(OR')_{(4-n-m)}(OR'')_m + mR'OH$$

Le nombre n étant 1 à 3, m 3 à 1.
Les cas les plus fréquents sont :

$$RSi(OR')_2(OR'')_1 \ ; \ RSi(OR')_1(OR'')_2$$
$$et \ R_2Si(OR')(OR'')$$

Selon la nature des molécules R, R' et R'', en particulier suivant le caractère et le nombre des groupes actifs qu'elles portent, les proportions relatives de R, R' et R'' dans le silicium cosmétique, suivant l'invention, peuvent varier largement. En général, mais non obligatoirement, la composition renferme 0,2 à 25 atomes de Si par mole de substance cosmétologiquement active R' ; plus souvent, ce rapport est de 0,3 à 10 atomes Si par mole de R'.

En ce qui concerne la molécule R'', dont la masse moléculaire peut varier considérablement, sa proportion peut être choisie dans les limites pondérales de 0,2 à 5 parties par partie de substance R' ou de R silanol, ces indications n'étant pas limitatives.

L'invention est illustrée non limitativement par les exemples, qui suivent, de fabrication et d'applications de divers silicium-cosmétique.

EXEMPLES 1 à 6

L'activité spécifique est étudiée suivant les différents R'. Dans ces silanols cosmétiques le caractère intracutané est dû à la molécule R''.

Des solutions aqueuses de différents R silanols et différents R' sont préparées toutes avec la même molécule R'', constituée par de l'élastine de masse moléculaire d'environ 80000, d'origine bovine, rendue préalablement soluble dans l'eau, par l'action d'une élastase, ou par hydrolyse acide ou alcaline, sans altération de la structure physique de l'élastine.

Les teneurs % en poids de la composition, en chacun des constituants R silanols, R', R'' sont données ci-après pour les exemples 1 à 6.

EXEMPLE 1

$R\text{-}Si(OH)_{4-n}$ = méthyl silane triol $CH_3Si(OH)_3$ ........ 0,5%

R' = théophylline

........0,45%

R'' = élastine solubilisée, structurée (décrite plus haut) ............... 0,5%

Dans cette composition, le rapport molaire silanol/R' ressort à 2,13 atomes de Si par mole de théophylline.

La composition présente, au point de vue cosmétologique, de bonnes propriétés amincissantes.

EXEMPLE 2

4

Méthyl silane triol ............................ 0,50%

R' = acide théophylline acétique

$$\text{H}_3\text{C} - \text{N} \quad \text{CH}_2\text{COOH}$$

...... 0,59%

R" = élastine solubilisée, structurée

(comme exemple 1) ...................0,50%

Il y a 2,14 atomes de Si par mole d'acide théophylline acétique.

Comme celle de l'exemple 1, cette composition exerce sur la peau une action lipolysante.

EXEMPLE 3

| Diméthyl silane diol $(CH_3)_2Si(OH)_2$ | 1,00% |
|---|---|
| R' = acide glycyrrhizique $C_{42}H_{62}O_{16}$ | 0,41% |
| R" = élastine solubilisée, structurée (comme dans l'exemple 1) | 0,75% |

La composition renferme 25 atomes de Si par mole d'acide glycyrrhizique ; ses propriétés anti-inflammatoires et restructurantes la rendent utile à l'application contre le coup de soleil.

EXEMPLE 4

| Méthyl silane triol | 0,70% |
|---|---|
| R' = glycine $NH_2CH_2COOH$ | 0,07% |
| R" = élastine solubilisée, structurée | 0,80% |

Il y a 8 atomes Si par mole de glycine.

La composition exerce une action cicatrisante très marquée.

EXEMPLE 5

Méthyl silane triol ........................... 0,70%

  R' = sphingomyéline .......................... 1,60%

$$CH_3(CH_2)_{12}CH=CH-CHOH-\overset{\overset{\displaystyle NHOCR}{|}}{CH}-CH_2-\overset{|}{O}$$
$$(R=C_{17}H_{35}) \quad HO(CH_3)_3NCH_2CH_2O--\overset{|}{\underset{\underset{\displaystyle O}{\downarrow}}{P}}-OH$$

  R'' = élastine solubilisée, structurée ........... 0,60%

Le rapport Si/R' est ici de 2,33 atomes Si par mole de sphingomyéline. La composition présente des propriétés antipelliculaires.

EXEMPLE 6

| | |
|---|---|
| Diméthyl silane diol $(CH_3)_2Si(OH)_2$ | 0,93% |
| R' = acide acétyl tyrosine | 2,19% |
| R'' = élastine solubilisée, structurée | 0,60% |

La solution contient 1 atome Si par mole d'acide acétyl tyrosine. Elle est active à 3,5% comme activateur de bronzage.

EXEMPLES 7 à 12

L'activité spécifique est la même pour tous ces silanol-cosmétiques, car on conserve le même R' tout en faisant varier R'', en respectant les divers groupes des composés organiques cités plus haut. Ces silanols-cosmétiques sont préparés suivant l'invention, comme dans les exemples de 1 à 6. La molécule R' est l'acétyltyrosine ; la molécule R'' est constituée par le collagène, l'esculoside, le cholestérol, sphingomyéline,pyrrolidon carboxylate de cuivre, tocophérol.

| EXEMPLE | Si = 0,15% R = 0,5 % | R' = 0,25 | R'' = 0,1 |
|---|---|---|---|
| 7 | $CH_3Si(OH)_3$ | | Polyphénol esculoside |
| 8 | " | | Pyrrolidone carboxylique de cuivre |
| 9 | $(CH_3)_2Si(OH)_2$ | | Collagène |
| 10 | $CH_3Si(OH)_3$ | acide acétyl tyrosine | Sphingomyéline |
| 11 | $(CH_3)_2Si(OH)_2$ | | Alcool oléique |
| 12 | $CH_3Si(OH)_3$ | | Cholestérol |

On procède au dosage du silicium dans la peau de rats mis en expérimentation au bout de 24 heures et 10 jours après applications d'un gel placébo lot témoin, d'un gel RR' lot I et d'un gel RR'R'' lot II, tels que défini plus haut pour étude comparative. Dans tous les cas, il a été appliqué 1g de gel sur 2 cm$^2$ de peau,pour les lots I et II, cela correspond à 500 microg de Si par application. Sur le lot témoin placébo on détermine la teneur en Si de la peau du rat, soit 114 ± 25 microg par g.

EP 0 289 366 B1

TABLEAU

| Dosage du Si dans la peau en $\mu$g/g | | | | | | | |
|---|---|---|---|---|---|---|---|
| Teneur en Si lot témoin | Dérivés | Quantité Si dosée Lot I | Surcharge en Si ou Si retenu dans la peau lot I | | Quantité Si dosée lot II | Surcharge en Si ou Si retenu dans la peau lot II | |
| 114±25 | 7 | 489±35 | 375±60 | 75% | 680±45 | 566±70 | 113% |
| | 8 | 474±23 | 360±48 | 72% | 595±34 | 480±59 | 96% |
| | 9 | 480±36 | 366±61 | 73% | 626±19 | 511±44 | 102% |
| | 10 | 465±43 | 351±68 | 70% | 609±38 | 495±63 | 99% |
| | 11 | 471±52 | 357±77 | 71% | 672±15 | 558±40 | 112% |
| | 12 | 492±26 | 378±51 | 76% | 612±25 | 498±50 | 99% |

On constate ainsi que les moyennes de quantité de Si retenues dans la peau, dans le cas des composés suivant l'invention R R' R'' sont voisines de 100% en tenant compte des écarts types tandis que celles qui correspondent au complexe RR' font apparaître un manque en Si de 40%.

EXEMPLE 13

Une composition pour crème régénératrice de la peau est préparée comme suit.

| Méthyl silane triol | 1% |
|---|---|
| R' = acide mannuronique | 0,625% |
| R'' = mucopolysaccharide | 0,5% |

Des résultats d'application de cette composition sont donnés plus loin, dans l'exemple 19.

EXEMPLE 14

L'acide acétyl tyrosine, en tant que substance capable d'exercer une action cosmétologique, a été employé sous la forme de la composition suivante :

Diméthyl silane diol $(CH_3)_2Si(OH)_2$ ............ 0,5%

R' = acide acétyl tyrosine

$$HO-\!\!\!\!\bigcirc\!\!\!\!-CH_2\underset{|}{CH}-COOH \qquad ............ 0,703\%$$
$$NH-COCH_3$$

R'' = polypeptides collagéniques de masses

moléculaires > à 40 000 ................ 1%

Le rapport Si/R' est ici de 1,59 atomes Si/mole d'acide acétyl tyrosine.
La composition constitue un bon activateur de bronzage.

EXEMPLE 14bis

R = $CH_3 Si(OH)_3$ R' = élastine R'' = sorbitol

EXEMPLE 15

Dans cette préparation, R'',qui fixe le silanol fonctionnalisé dans la peau, est constitué par des nucléoprotéines. La composition comprend :

7

| Méthyl silane triol | 0,5% |
|---|---|
| R' = acide lactique CH$_3$CHOHCOOH | 0,489% |
| R'' = nucléoprotéines | 1,5% |

Le rapport molaire Si/acide lactique = 0,98.
La composition exerce une action hydratante sur la peau.

APPLICATIONS DE COMPOSITIONS SUIVANT L'INVENTION

EXEMPLE 16

Application du silanol cosmétique de l'exemple 2 dans une crème amincissante, hydrodispersible.

La crème, à 5% du silanol cosmétique est appliquée quotidiennement sur les zones adipeuses de 10 patientes,en 50 séances de massages. Des résultats obtenus comparativement à un placebo et à une solution à 6% de théophylline acétique (ce qui donne un rapport de 1 à 10), on tire les moyennes suivantes :

| | Silanol cosmétique à 5% | Placébo | Acide théophylline acétique 6% |
|---|---|---|---|
| Taille | -3,5 = 5% | - 0,4 | -1,2 = 1,7 % |
| Hanches | -4,4 = 4,9% | - 0,6 | -1 = 1,1 % |
| Cuisses | -1,7 = 3,8% | + 0,1 | -0,2 = 0,4% |
| Genoux | -0,6 | 0 | 0 = 0 |

Il s'en suit donc que le silicium cosmétique suivant l'invention active fortement l'amincissement.

D'autre part, on effectue 5 cultures d'adipocytes humains, surchargés d'acides gras marqués :

(A) - culture témoin sans additif ;

(B) - culture additionnée de 1 mg par ml de culture d'une solution à 0,45% de théophylline ;

(C) - culture additionnée de 1 mg par ml de culture d'une solution à 0,59% d'acide théophylline acétique ;

(D) - culture additionnée de 1 mg par ml de culture du silanol cosmétique de l'exemple 1 ;

(E) - culture additionnée de 1 mg par ml de culture du silanol cosmétique de l'exemple 2.

L'activité lipolytique a été trouvée double, dans les cultures (D) et (E), par rapport au témoin (A), tandis qu'elle ne s'est accrue que d'environ 20% pour les cultures (B) et (C). Le silanol cosmétique D et E a donc exercé une influence positive.

EXEMPLE 17

Application de la composition de l'exemple 3 contre l'érythème solaire.

Une lotion gélifiée,à 6% de la composition de l'exemple 3, est appliquée dans plusieurs cas de "coup de soleil", parallèlement à une lotion similaire à 6% d'une solution à 0,75% d'acide glycyrrhyzique,sans silanol ni R''. On constate que le silanol cosmétique de l'exemple 3 fait disparaître rapidement la douleur puis l'intensité de la rougeur, et évite toute desquamation, si elle est appliquée durant 3 jours, En l'absence de silanol-cosmétique, en mesurant l'intensité relative de l'érythème on obtient les résultats suivants.

| | Silanol cosmétique | acide glycyrrhizique | Placébo |
|---|---|---|---|
| Douleur | pas | assez forte sans chaleur | forte avec chaleur |
| Intensité érythémateuse | 1 | 3,5 | 5 |
| Desquamation | pas | faible | importante |

EXEMPLE 18

Application du silanol cosmétique de l'exemple 14 bis qui met en évidence l'action régénératrice.

Après avoir pratiqué une incision sur la peau du dos du rat, on laisse cicatriser 7 jours, puis on applique le produit, ainsi que le placébo pendant 17 jours. L'étude histologique de la cicatrice permet d'observer un épiderme restructuré, sans bourrelet, comparable à l'épiderme sain; la zone sous-épidermique est régénérée, en comparaison avec un témoin placébo où apparaît un bourrelet cicatriciel avec un épiderme hyperplasié. Le tissu sousjacent est encore anarchique et désorienté.

EXEMPLE 19

Application de la composition de l'exemple 13.

On utilise une émulsion cosmétique à 6% de ladite composition, en tant que crème régénératrice.

Les observations ont été faites sur des femmes vieillissantes, après application durant deux mois. L'appréciation de l'efficacité du produit se fait par les tests d'élasticité,c'est-à-dire mesure comparative de la rétractabilité de la peau après étirement sous une force déterminée. L'amélioration de la rétractabilité avant et après traitement s'est améliorée d'une façon significative.

D'autre part, on a constaté que, 6 heures après l'application de la composition, 96% du silanol mis en oeuvre reste dans la peau ; par contre , il n'en reste que 75% lorsque la composition utilisée ne contient pas de mucopolysaccharide. Cela caractérise bien le rôle de celui-ci de fixation de la substance active dans la peau.

EXEMPLE 20

Application du silanol cosmétique de l'exemple 14, en tant qu'activateur de bronzage.

Pour cela on utilise une préparation cosmétique à 7% de la solution selon l'exemple 14. Des tests comparatifs sont réalisés sur un même sujet de peau de type 1, par exposition aux U.V. A et U.V. B. Un hâle est observé sur la surface de la peau traitée, alors que sur la peau exposée, mais non traitée, il y a un érythème indice 2. Sur une peau de type 2, on observe un hâle intense sur la surface traitée tandis que la peau exposée, sans traitement préalable, était légèrement rosée.

On observe des résultats plus fugaces lorsque la composition ne contenait pas de polypeptides collagéniques. Cela montre l'intérêt d'activateur de bronzage que présente la composition particulière,suivant l'exemple 14, pour les peaux de type 1 ou 2 subissant des expositions intensives de courte durée.

EXEMPLE 21

Application de la présente invention à un conditionneur pour cheveux dont la composition peut être :

| Méthylsilane triol | de 0,1 à 5% |
| Elastine solubilisée et structurée | de 1 à 10% |
| Polyester d'acide gras | de 0,1 à 5% |

La variation des quantités indiquées permet de modifier la qualité du conditionneur pour cheveux ou de la laque, c'est-à-dire degrés de fixation sur le cheveu, gonflement, aspect luisant. La solution peut être aqueuse ou alcoolique et permet d'obtenir une laque biologique ou un conditionneur pour cheveux par gainage des cheveux.

**Revendications**

1. Procédé de préparation d'une solution aqueuse pour application cutanée ou capillaire, par mélange d'une solution d'un silanol avec une matière cosmétique, caractérisé en ce que l'on ajoute à la solution une substance qui empêche le passage du silicium, appartenant au silanol utilisé, dans les tissus sousjacents de la peau.

2. Procédé suivant la revendication 1, caractérisé en ce que les proportions des composants sont tels qu'il y ait 0,2 à 25 atomes de Si par mole de matière cosmétique et 0,2 à 5 parties en poids de substance empêchant le passage de Si dans les tissus sous-jacents, par partie en poids de ladite matière cosmétique.

3. Procédé suivant la revendication 1 ou 2, caractérisé en ce que la solution est préparée à partir d'un produit formé par transalcoxylation d'un siloxane, comprenant la matière cosmétique porteuse d'un groupe OH, $NH_2$ ou/et COOH, et ladite substance empêchant le passage de Si dans les tissus sous-jacents, comportant des groupes OH, $NH_2$, ou/et COOH.

4.  Produit pour l'application cutanée ou capillaire, qui contient une matière cosmétique (R') et un composé organique du Si, caractérisé en ce qu'il comprend, en outre, une substance (R'') qui empêche le passage du Si organique dans les tissus sous-jacents de la peau, le produit comportant un complexe moléculaire de formule générale :

$R_n Si(OR')_m (OR'')_p$

où $n + m + p = 4$, R est un alkyle, alcényle ou aryle, n est un nombre de 1 à 3, R' est la molécule d'une matière cosmétique et R'' celle d'une substance absorbable par la peau, portant une ou plusieurs des fonctions alcool, phénol, acide, amino et amino-acide, et qui empêche le Si organique de passer dans les tissus sous-jacents.

5.  Produit suivant la revendication 4, caractérisé en ce que ladite matière (R') est la théophylline, un dérivé de la théophylline, l'acide acétyl tyrosine, l'acide glycyrrhizique, la glycine, l'acide lactique, l'hydroxyproline, la sphingomyéline, l'acide mannuronique, ou parahydroxycinnamique.

6.  Produit suivant une des revendications 4 ou 5, caractérisé en ce que la substance (R'') est un protide, lipoprotide, mucopolysaccharide ou nucléoprotéine.

7.  Produit suivant une des revendications 4 à 6, caractérisé en ce que la matière, qui empêche le composé organique du Si de passer dans des tissus sous-jacents à la peau, est une protéine naturelle, ayant conservé sa structure naturelle, en particulier élastine, après avoir subi une hydrolyse partielle.

8.  Produit suivant une des revendications 4 ou 5, caractérisé en ce que la substance (R'') est un polyphénol seul ou sous la forme d'hétéroside, un composé stérolique, un alcool gras ou un oligoélément métallique.

## Claims

1.  A method for producing an aqueous solution for skin or hair application, by missing a silanol solution with a cosmetic material, characterized by adding to the solution a substance preventing the silicone belonging to the silanol used from going into the underlying tissues of the skin.

2.  The method of claim 1, characterized by proportions of components being such that there are 0.25 to 25 atoms of Si, per mole of cosmetic material, and 0.2 to 5 parts in weight of substance preventing Si from going into the underlying tissues by part in weight of said cosmetic material.

3.  The method of claim 1 or 2, characterized in that the solution is prepared from a product formed by transal koxylation of a siloxane, comprising the cosmetic material with a group, OH, $NH_2$ and/or COOH, and said substance preventing Si from going into the underlying tissues, comprising groups OH, $NH_2$ and/or COOH.

4.  A product intended for skin or hair application, comprising a cosmetic material (R') and an organic compound of Si, characterized by further comprising a substance (R'') which prevents organic Si from going into the underlying tissues of the skin, the product comprising a molecular complex having the general formula :

$R_n Si(OR')_m (OR'')_p$

where

$n + m + p = 4$,

R is alkyl, alkenyl or aryl

n is a number from 1 to 3

R' is the molecule of a cosmetic material, and

R'' is the molecule of a substance which can be absorbed by the skin, comprises one or several alcohol, phenol, acid, amine or aminoacid groups, and prevents organic Si from going into the underlying tissues.

5. The product of claim 4, characterized in that said material (R') is theophylline, a theophylline derivative, acetyltyrosine acid, glycyrrhysic acid, glycine, lactic acid, hydroxyproline, sphingomyeline, mannuronic acid or parahydroxycinnamic acid.

6. The product of claim 4 or 5, characterized in that said substance (R'') is a protide, lipoprotide, mucopolysaccharide or nucleoprotein.

7. The product of one of claims 4 to 6, characterized in that the material preventing the organic compound of Si from going into the underlying tissues of the skin is a natural protein which has kept its natural structure, in particular elastine, after a partial hydrolysis.

8. The product of one of claims 4 or 5, characterized in that the substance (R'') is a polyphenol alone or in the form of a heteroside, a sterolic compound, a fatty alcohol or a metallic oligoelement.

## Patentansprüche

1. Verfahren zur Herstellung einer wässrigen Lösung zur Anwendung auf die Haut oder das Haar durch Mischen einer Lösung eines Silanols mit einem kosmetischen Material, dadurch gekennzeichnet, dass man der Lösung eine Substanz zusetzt, die den Eintritt des Siliziums, das dem verwandten Silanol angehört, in die unter der Haut liegenden Gewebe verhindert.

2. Verfahren nach Anspruch 1, dadurch gekennzeichnet, dass die Anteile der Bestandteile derart sind, daß 0,2 bis 25 Si-Atome pro Mol des kosmetischen Materials und 0,2 bis 5 Gew.-Teile der Substanz, die den Eintritt des Si in die darunterliegenden Gewebe verhindert, pro Gew.-Teil des kosmetischen Materials vorhanden sind.

3. Verfahren nach Anspruch 1 oder 2, dadurch gekennzeichnet, dass die Lösung ausgehend von einem Produkt erzeugt wird, das durch Transalkoxylierung eines Siloxans gebildet ist, welches das eine OH, $NH_2$- und/oder COOH-Gruppe aufweisende kosmetische Material und die den Durchgang des Si in das darunterliegende Gewebe hindernde Substanz, die OH-, $NH_2$- und/oder COOH-Gruppen aufweist, enthält.

4. Produkt zur Anwendung auf die Haut oder das Haar, welches ein kosmetisches Material (R') und eine organische Si-Verbindung enthält, dadurch gekennzeichnet, dass sie unter anderem eine Substanz (R'') umfasst, die den Durchgang von organischem Si in die darunterliegenden Gewebe der Haut hindert, wobei das Produkt einen molekularen Komplex der allgemeinen Formel enthält:

$$R_nSi(OR')_m(OR'')_p$$

worin n + m + p = 4, R ein Alkyl, Alkenyl oder Aryl ist, n eine Zahl von 1 bis 3 ist, R' ein Molekül eines kosmetischen Materials ist und R'' das einer durch die Haut absorbierbaren Substanz, die eine oder mehrere Alkohol-, Phenol-, Säure-, Amino- und Aminosäurenfunktionen aufweist und die das organische Si daran hindert, in die darunterliegende Gewebe einzutreten.

5. Produkt nach Anspruch 4, dadurch gekennzeichnet, dass das Material (R') Theophyllin, ein Theophyllinderivat, Acetyltyrosinsäure, Glycyrrhizinsäure, Glycin, Milchsäure, Hydroxyprolin, Sphingomyelin, Mannuronsäure oder Parahydroxyzinnsäure ist.

6. Produkt nach einem der Ansprüche 4 oder 5, dadurch gekennzeichnet, dass die Substanz (R'') ein Proteid, Lipoproteid, Mucopolysaccharid oder Nukleoprotein ist.

7. Produkt nach einem der Ansprüche 4 bis 6, dadurch gekennzeichnet, dass das Material, die die organische Si-Verbindung am Eintritt in die darunterliegenden Gewebe der Haut hindert, ein natürliches Protein ist, das seine natürliche Struktur bewahrt hat, insbesondere Elastin, das eine partielle Hydrolyse eingegangen ist.

8. Produkt nach einem der Ansprüche 4 oder 5, dadurch gekennzeichnet, dass die Substanz (R'') ein Polyphenol allein oder in Heterosidform, eine Sterolinverbindung, ein Fettalkohol oder ein metallisches Oligoelement ist.